# EUROPÄISCHE PATENTSCHRIFT

(11) **EP 1 889 046 B1**
(45) Veröffentlichungstag und Bekanntmachung des Hinweises auf die Patenterteilung: **04.08.2010**
(21) Anmeldenummer: 06753999.9
(22) Anmeldetag: 31.05.2006
(51) Int. Cl.: G01N 29/02

(54) **VERFAHREN UND VORRICHTUNG ZUM ÜBERWACHEN EINER STRÖMENDEN FLÜSSIGKEIT MITTELS ULTRASCHALL AUF DAS VORHANDENSEIN VON LUFT**
METHOD AND DEVICE FOR MONITORING A FLOW OF LIQUID FOR THE PRESENCE OF AIR BY MEANS OF ULTRASOUND
PROCEDE ET DISPOSITIF POUR SURVEILLER PAR ULTRASONS LA PRESENCE D'AIR DANS UN ECOULEMENT LIQUIDE

(30) Priorität: 03.06.2005 DE 102005025500
(43) Veröffentlichungstag der Anmeldung: 20.02.2008
(73) Patentinhaber: Fresenius Medical Care Deutschland GmbH, 61352 Bad Homburg v.d.H. (DE)
(72) Erfinder: SCHNEIDER, Jochen, 97537 Wipfeld (DE); WAMSIEDLER, Ralf, 97469 Gochsheim-Weyer (DE)
(74) Vertreter: Luderschmidt, Schüler & Partner
(86) Internationale Anmeldenummer: PCT/EP2006/005171
(87) Internationale Veröffentlichungsnummer: WO 2006/128683

(56) Entgegenhaltungen:
- EP-A- 1 182 452
- US-A- 4 607 520
- US-A- 5 824 881

## Beschreibung

Die Erfindung bezieht sich auf ein Verfahren zum Überwachen einer strömenden Flüssigkeit auf das Vorhandensein von Luft, insbesondere des in einem extrakorporalen Blutkreislauf einer extrakorporalen Blutbehandlungsvorrichtung strömenden Blutes, sowie ein Verfahren zur extrakorporalen Blutbehandlung mit einem extrakorporalen Blutkreislauf, wobei das Vorhandensein von Luft in dem extrakorporalen Blutkreislauf überwacht wird. Darüber hinaus betrifft die Erfindung eine Vorrichtung zum Überwachen einer strömenden Flüssigkeit, insbesondere des in einem extrakorporalen Kreislauf strömenden Blutes, auf das Vorhandensein von Luft, sowie eine Vorrichtung zur extrakorporalen Blutbehandlung mit einer Vorrichtung zur Überwachung des in dem extrakorporalen Kreislauf der Blutbehandlungsvorrichtung strömenden Blutes auf das Vorhandensein von Luft.

Es sind verschiedene Verfahren zur extrakorporalen Blutbehandlung bekannt, bei denen das Blut des Patienten in einem extrakorporalen Blutkreislauf durch eine Blutbehandlungseinheit strömt. Eine der wesentlichen Komplikationen bei der extrakorporalen Blutbehandlung, beispielsweise Hämodialyse oder Hämofiltration, stellt die Möglichkeit des Eindringens von Luft in den extrakorporalen Blutkreislauf dar. Zum Abscheiden mitgeführter Luftblasen aus dem Blut dienen die bekannten Tropfkammern, die im venösen Zweig des extrakorporalen Blutkreislaufs angeordnet sind.

Die bekannten Tropfkammern fangen die Luftblasen mit hoher Sicherheit ab. Dennoch besteht grundsätzlich die Gefahr, dass Luftblasen dem Patienten intravenös infundiert werden. Zur weiteren Erhöhung der Sicherheit verfügen die Blutbehandlungsvorrichtungen daher über Luftdetektoren, an deren Funktionssicherheit sehr hohe Anforderungen gestellt werden. Mit den Luftdetektoren mussen sich nicht nur größere Luftblasen, sondern auch kleine Luftblasen mit hoher Sicherheit erkennen lassen. Während beim Auftreten einer größeren Luftblase die Behandlung sofort unterbrochen und Alarm gegeben wird, kann eine gewisse Anzahl von kleineren Einzelblasen noch toleriert werden. Allerdings darf das Gesamtvolumen der im Blut eingeschlossenen Luft einen vorgegebenen Grenzwert nicht überschreiten.

Die EP 1 182 452 A2 beschreibt eine Vorrichtung zur Erkennung von Luftblasen in strömenden Flüssigkeiten, die auf einer Ultraschallmessung beruht. Die Luftblasen werden an der Dämpfung der eine Messstrecke durchlaufenden Ultraschallsignale erkannt. Dabei ist die Höhe der Dämpfung ein Maß für die Größe der Luftblasen.

Die bekannte Überwachungsvorrichtung verfügt über einen Ultraschallsender zum Einkoppeln der Ultraschallsignale mit einem vorgegebenen Pegel in die strömende Flüssigkeit und einen Ultraschall-Empfänger zum Empfangen der aus der strömenden Flüssigkeit austretenden Ultraschallsignale. Das Ausgangssignal des Ultraschall-Empfängers wird mit einem vorgegebenen Grenzwert verglichen. Wenn das Ultraschallsignal kleiner als der Grenzwert ist, wird auf eine große Luftblase geschlossen, während bei einem Ultraschallsignal, das größer als oder gleich dem Grenzwert ist, eine kleine Luftblase angenommen wird.

Bei einer großen Luftblase gibt die Überwachungsvorrichtung sofort einen Alarm. Das Auftreten kleinerer Luftblasen hingegen ist nicht sofort mit einem Alarm verbunden. Ein Alarm wird nur dann gegeben, wenn die in der strömenden Flüssigkeit befindliche Luft ein kritisches Gesamtvolumen überschreitet. Das in der Flüssigkeit eingeschlossene Luftvolumen wird aus der Anzahl der kleinen Luftblasen und der Flussrate der strömenden Flüssigkeit berechnet. Hierzu werden die kleinen Luftblasen gezählt, die mit der Ultraschallmessung erkannt werden.

Die aus der EP 1 182 452 A2 bekannte Überwachungsvorrichtung ist insbesondere zur Überwachung des extrakorporalen Blutkreislaufs einer Blutbehandlungsvorrichtung bestimmt.

Die US 4 651 555 beschreibt ein Verfahren zum Überwachen einer strömenden Flüssigkeit auf das Vorhandensein von Partikeln oder Gasblasen, bei dem Schallsignale in die Flüssigkeitsströmung eingekoppelt und die aus der Flüssigkeit austretenden Schallsignale empfangen werden. Die empfangenen Schallsignale werden mit einem Grenzwert verglichen, wobei auf das Vorhandensein einer Luftblase dann geschlossen wird, wenn die empfangenen Schallsignale kleiner als der Grenzwert sind. Der Grenzwert, mit dem die Schallsignale verglichen werden, wird auf der Grundlage eines Mittelwertes bestimmt, der aus den empfangenen Schallsignalen gebildet wird.

Ein Überwachungsverfahren für strömende Flüssigkeiten, das auf der Auswertung der Dämpfung von Schallsignalen beruht, die eine Messstrecke durchlaufen, ist auch aus der US 4 487 601 bekannt.

Die EP 1466 637 A2 beschreibt eine Vorrichtung zum Erkennen von Luftblasen auf der Grundlage einer Ultraschallmessung, bei der die Einhüllende des empfangenen Signals zur Bestimmung des Volumens der Luftblasen analysiert wird. Wenn das Gesamtvolumen der Luftblasen innerhalb einer bestimmten Zeitdauer einen vorgegebenen Grenzwert überschreitet, wird ein Alarm ausgelöst.

Die US 5 824 881 beschreibt eine Vorrichtung zur Detektion eines Fluids oder Gases in einem Fluidsystem, insbesondere in medizinischen Transfusionsanordnungen. Mittels der Amplitude von Ultraschallsignalen in aufeinanderfolgenden Zeitintervallen wird auf das Vorhandensein von Luft geschlossen.

Der Erfindung liegt die Aufgabe zugrunde, ein Verfahren zum Überwachen einer strömenden Flüssigkeit auf das Vorhandensein von Luft anzugeben, das eine Unterscheidung zwischen kleinen und großen Luftblasen und die Erkennung unterschiedlicher Störfälle mit hoher Sicherheit erlaubt. Eine weitere Aufgabe der Erfindung ist, ein Verfahren zur extrakorporalen Blutbehandlung mit einem extrakorporalen Blutkreislauf anzugeben, bei dem mit hoher Sicherheit kleine und große Luftblasen im extrakorporalen Kreislauf erkannt und zwischen unterschiedlichen Störfällen unterschieden werden kann. Die Lösung dieser Aufgaben erfolgt erfindungsgemäß mit den Merkmalen der Patentansprüche 1 und 7.

Darüber hinaus liegt der Erfindung die Aufgabe zugrunde, eine Vorrichtung zum Überwachen einer strömenden Flüssigkeit auf das Vorhandensein von Luft und eine extrakorporale Blutbehandlungsvorrichtung mit einer derartigen Überwachungsvorrichtung bereitzustellen. Die Lösung dieser Aufgaben erfolgt mit den Merkmalen der Ansprüche 8 und 14.

Das Funktionsprinzip des erfindungsgemäßen Verfahrens und der erfindungsgemäßen Vorrichtung beruht darauf, dass zur Unterscheidung zwischen kleinen und großen Luftblasen nicht allein die Dämpfung eines einzigen Ultraschall-Impulses herangezogen wird, sondern die Amplitude jedes der in einer kontinuierlichen Folge von Zeitintervallen empfangenen Ultraschallsignale mit einem vorgegebenen Referenzpegel verglichen wird, wobei die Anzahl der aufeinanderfolgenden Zeitintervalle ermittelt wird, in denen die Amplitude des in dem jeweiligen Zeitintervall empfangenen Ultraschallsignals kleiner als der vorgegebene Referenzpegel ist. Die ermittelte Anzahl der aufeinanderfolgenden Zeitintervalle, in denen die Amplitude des empfangenen Ultraschallsignals kleiner als der vorgegebene Referenzpegel ist, wird wiederum mit einem Grenzwert verglichen, der in Abhängigkeit von der Flussrate der strömenden Flüssigkeit festgelegt wird. Auf einen Störfall wird dann geschlossen, wenn die Anzahl der aufeinanderfolgenden Zeitintervalle größer als der festgelegte Grenzwert ist. In diesem Fall wird davon ausgegangen, dass eine große Luftblase vorliegt.

Der Grenzwert, mit dem die Anzahl der aufeinanderfolgenden Zeitintervalle verglichen wird, kann in Versuchen empirisch ermittelt werden, wobei verschiedene Sicherheitskriterien Berücksichtigung finden können. Mit zunehmender Flussrate der strömenden Flüssigkeit wird der Grenzwert kleiner.

Auf einen Störfall wird also nur dann geschlossen, wenn in einer bestimmten Anzahl von aufeinanderfolgenden Zeitintervallen ein bestimmtes Volumen an Luft erkannt wird. Folglich wird ein Störfall dann nicht angenommen, wenn zwar die Anzahl der Zeitintervalle, in denen ein bestimmtes Volumen an Luft erkannt wird, den kritischen Grenzwert überschreitet, die Zeitintervalle aber nicht kontinuierlich aufeinander folgen. Somit kommt es nicht nur auf die Gesamtzahl der Ereignisse an.

Für den Fall, dass die Anzahl der aufeinanderfolgenden Zeitintervalle, in denen ein bestimmte Volumen an Luft erkannt wird, kleiner als oder gleich dem festgelegten Grenzwert ist, wird darauf geschlossen, dass nicht eine große Luftblase bzw. ein großes Luftvolumen, sondern nur eine oder mehrere kleine Luftblasen bzw. ein kleines Luftvolumen (Einzelblasen) in der Flüssigkeit eingeschlossen ist. Dieses kleine Luftvolumen kann dann toleriert werden, wenn es eine kritische Größe nicht überschreitet.

Anstelle der Amplitude des Ultraschallsignals kann auch jede mit der Amplitude korrelierende Größe für den Vergleich herangezogen werden. So kann beispielsweise die maximale Amplitude des Signals oder nur die Amplitude der oberen oder unteren Halbwelle ausgewertet werden. Bei impulsförmigen Signalen beispielsweise, deren Amplitude mit der Zeit abnimmt, wird vorzugsweise die maximale Signalamplitude bestimmt. Dabei handelt es sich bei der Amplitude immer um eine positive Größe, auch wenn der absolute Wert des Signals zu einem bestimmten Zeitpunkt negativ sein sollte.

Bei einer bevorzugten Ausführungsform der Erfindung wird die Anzahl der Ereignisse ermittelt, bei denen ein kleines Luftvolumen erkannt wird, wobei die Anzahl dieser Ereignisse mit einem vorgegebenen Referenzwert verglichen wird. Auf einen Störfall wird dann geschlossen, wenn die Anzahl der Ereignisse größer als der vorgegebene Referenzwert ist. In diesem Fall hat das gesamte in der Flüssigkeit eingeschlossene Luftvolumen die kritische Größe überschritten. Vorzugsweise wird beim Erkennen des einen und/oder anderen Störfalls ein erstes Alarm- und/oder Steuersignal erzeugt. In einer extrakorporalen Blutbehandlungsvorrichtung wird beim Erzeugen eines Alarm- und/oder Steuersignals Alarm gegebenen und/oder die Behandlung unterbrochen. Hierzu verfügen die bekannten Blutbehandlungsvorrichtungen über geeignete Einrichtungen.

Eine weitere bevorzugte Ausführungsform der Erfindung sieht vor, dass unterschiedliche Signale erzeugt werden, wenn die Amplitude des empfangenen Ultraschallsignals kleiner bzw. größer oder gleich dem vorgegebenen Referenzpegel ist, wobei die zeitliche Folge der unterschiedlichen Signale gespeichert wird. Damit ist es möglich, eine differenzierte Aussage über die Größe der in der strömenden Flüssigkeit befindlichen Luftblase sowie deren Verteilung zu treffen:

Grundsätzlich ist es ausreichend, wenn die Amplitude des empfangenen Ultraschallsignals mit nur einem vorgegebenen Referenzpegel verglichen wird, um zwischen einem größeren und einem kleineren Luftvolumen unterscheiden zu können. Es ist aber auch möglich, mehrere vorgegebene Referenzpegel zu definieren, um zwischen Luftblasen unterschiedlicher Volumina differenzieren zu können.

Als besonders vorteilhaft hat sich erwiesen, wenn die beiden Signale, die beim Über- bzw. Unterschreiten des Grenzwertes erzeugt werden, pulsweitenmodulierte Signale (PWM-Signale) unterschiedlicher Pulsweite sind. Die PWM-Signale lassen sich mit hoher Störsicherheit übertragen, da eine klare Unterscheidung zwischen einem pulsweitenmodulierten Signal und einem Störsignal möglich ist. So kann beispielsweise auch eine Leitungsunterbrechung, die zum Ausbleiben eines Signals führt, sicher erkannt werden.

Die Länge der Zeitintervalle, in denen die Ultraschallsignale ausgewertet werden, d.h. die Abtastfrequenz, ist so zu bemessen, dass eine ausreichende zeitliche Auflösung möglich ist. In der Praxis sollte die Abtastfrequenz bei 5 kHz (T = 200µs) liegen. Da der Maschinenzyklus der bekannten Blutbehandlungsvorrichtungen mit T = 50ms deutlich über der zeitlichen Abfolge der Messzyklen liegt, werden die ersten und zweiten Signale, vorzugsweise PWM-Signale zu Signalblöcken zusammengefasst und die Signalblöcke vor der Auswertung zwischengespeichert. Nach der Zwischenspeicherung können die Signalblöcke dann im Takt der Maschine ausgewertet werden.

Die für die Auswertung der Signale erforderlichen Komponenten sind in den bekannten Blutbehandlungsvorrichtung im Allgemeinen vorhanden. Hierzu zählen beispielsweise ein Computer, mit dem die erforderlichen Vergleiche und Berechnungen nach entsprechender Programmierung durchgeführt werden können.

Im Folgenden wird ein Ausführungsbeispiel der Erfindung unter Bezugnahme auf die Zeichnungen näher erläutert.

Es zeigen:
- Figur 1: Die wesentlichen Komponenten einer Blutbehandlungsvorrichtung mit einem extrakorporalen Blutkreislauf in stark vereinfachter schematischer Darstellung zusammen mit einer Vorrichtung zum Überwachen des im Blutkreislauf strömenden Blutes auf das Vor- handensein von Luftblasen,
- Figur 2: die pulsweitenmodulierten Signale für Flüssigkeit und Luft,
- Figur 3: die Signalfolge der pulsweitenmodulierten Signale,
- Figur 4: eine Tabelle, in der der Grenzwert für die Anzahl der aufeinander- folgenden Zeitintervalle, in denen der Pegel des empfangenen Ult- raschallsignals kleiner als der vorgegebene Referenzpegel ist, in Abhängigkeit von der Flussrate der strömenden Flüssigkeit darge- stellt ist und
- Figur 5: den festgelegten Grenzwert als Funktion der Flussrate der strömen- den Flüssigkeit gemäß der Tabelle von Figur 4.

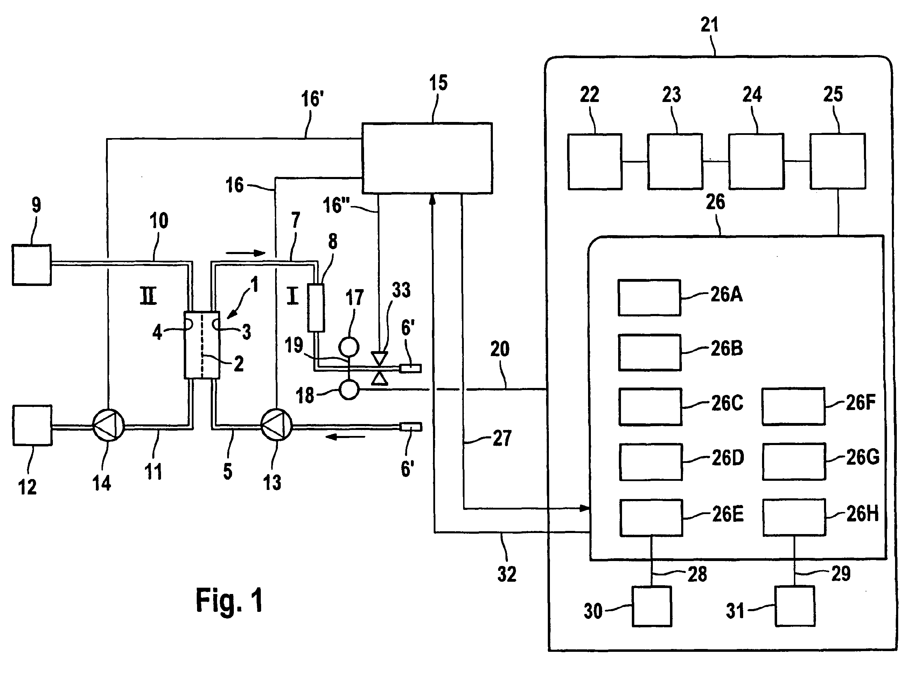

Figur 1 zeigt die wesentlichen Komponenten der Blutbehandlungsvorrichtung zusammen mit der Überwachungsvorrichtung. Die Blutbehandlungsvorrichtung, beispielsweise eine Hämodialysevorrichtung, weist einen Dialysator 1 auf, der durch eine semipermeable Membran 2 in eine Blutkammer 3 und eine Dialysierflüssigkeitskammer 4 getrennt ist. Der Einlass der Blutkammer ist mit einem Ende einer Blutzuführleitung 5 verbunden, während der Auslass der Blutkammer 3 mit dem einen Ende einer Blutabführleitung 7 verbunden ist, in die eine Tropfkammer 8 geschaltet ist. Die anderen Enden der Blutzuführ- und -abführleitung 5, 7 sind mit einer arteriellen bzw. venösen Nadel 6, 6' verbunden. Zwischen Tropfkammer 8 und venöser Nadel 6' ist eine elektromagnetisch betätigbare venöse Schlauchklemme 33 zum Abklemmen der Blutabführleitung 7 angeordnet. Die Blutzuführ- und -abführleitung 5, 7 stellen zusammen mit der Blutkammer 3 des Dialysators 1 den extrakorporalen Blutkreislauf I der Hämodialysevorrichtung dar.

Das Dialysierflüssigkeitssystem II der Hämodialysevorrichtung umfasst eine Einrichtung 9 zur Aufbereitung der Dialysierflüssigkeit, von der eine Dialysierflüssigkeitszuführleitung 10 abgeht, die zu der Dialysierflüssigkeitskammer 4 des Dialysators 1 führt. Von der Dialysierflüssigkeitskammer 4 geht eine Dialysierflüssigkeitsabführleitung 11 ab, die zu einem Auslass 12 führt.

In der Blutzuführleitung 5 ist eine Blutpumpe 13 angeordnet, während in der Dialysierflüssigkeitsabführleitung 11 eine Dialysierflüssigkeitspumpe 14 angeordnet ist. Blutpumpe 6 und Dialysierflüssigkeitspumpe 14 fördern während der Blutbehandlung Blut im extrakorporalen Blutkreislauf I bzw. Dialysierflüssigkeit im Dialysierflüssigkeitssystem II.

Die Hämodialysevorrichtung umfasst eine zentrale Steuereinheit 15, die über Steuerleitungen 16, 16', 16" mit der Blutpumpe 13 bzw. Dialysierflüssigkeitspumpe 14 bzw. Schlauchklemme 33 verbunden ist.

Darüber hinaus verfügt die Hämodialysevorrichtung über eine Vorrichtung zum Überwachen des im extrakorporalen Blutkreislaufs I strömenden Blutes auf das Vorhandensein von Luftblasen. Diese Überwachungsvorrichtung ist bei der beschriebenen Ausführungsform Bestandteil der Hämodialysevorrichtung, sie kann aber auch eine separate Baugruppe bilden.

Die Überwachungsvorrichtung weist einen Ultraschall-Sender 17 und einen Ultraschall-Empfänger 18 auf. Sender 17 und Empfänger 18 sind zu beiden Seiten der Blutrückführleitung 7 stromab der Tropfkammer 8 auf der Messstrecke 19 einander gegenüberliegend angeordnet.

Der Ultraschall-Sender 17 erzeugt kontinuierlich Ultraschallsignale im Impulsbetrieb in bestimmten Zeitintervallen von beispielsweise T = 200µs (f=5 kHz). Die Ultraschallsignale, die eine vorgegebene maximale Amplitude haben, durchlaufen die Messstrecke 19 und werden von dem Ultraschall-Empfänger 18 empfangen. Der Empfänger 18 erzeugt elektrische Ausgangssignale, die proportional zu der Amplitude der empfangenen Ultraschallsignale sind. Die Ausgangssignale des Empfängers 18 werden über eine Signalleitung 20 der Auswerteinheit 21 zugeführt.

Die Auswerteinheit 21 verfügt über eine Schaltung 22 zur Signalaufbreitung, einen Komparator 23, eine Schaltung 24 zur Pulsweitenmodulation PWM, einen Zwischenspeicher 25 und eine Schaltung 26 zur Signalauswertung. Die einzelnen Komponenten können in analoger oder digitaler Technik aufgebaut sein. Vorteilhafterweise sind die Komponenten Bestandteil der Hard- bzw. Software der Blutbehandlungsvorrichtung.

Die kontinuierliche Folge der Ausgangssignale des Ultraschall-Empfängers 18 werden der Schaltung 22 zugeführt, in der die Signale zur weiteren Auswertung aufbereitet werden. Die aufbereiteten Signale werden daraufhin dem Komparator 23 zugeführt, der die maximale Amplitude jedes Signals mit einem vorgegebenen Referenzpegel vergleicht. Dabei ist die Amplitude oder eine damit korrelierende Größe ein Maß für die Dämpfung des Signals, die von dem Volumen der im Blut eingeschlossenen Luft abhängig ist. Da es sich um impulsförmige Signale handelt, deren Amplitude mit der Zeit abnimmt, wird als der auszuwertende Signalpegel die maximale Amplitude des Signals ermittelt. Bei dem vorgegebenen Referenzpegel handelt es sich um eine Größe, die für ein bestimmtes Luftvolumen, das relativ klein ist, charakteristisch ist.

Der Komparator 23 vergleicht jedes der in der kontinuierlichen Folge von Zeitintervallen empfangenen Signale mit dem vorgegebenen Referenzpegel, wobei darauf geschlossen wird, dass das bestimmte Volumen an Luft im Blut eingeschlossen ist, wenn die maximale Amplitude des Signals kleiner als der vorgegebene Referenzpegel ist. Ansonsten wird darauf geschlossen, dass das bestimmte Volumen an Luft nicht vorhanden ist.

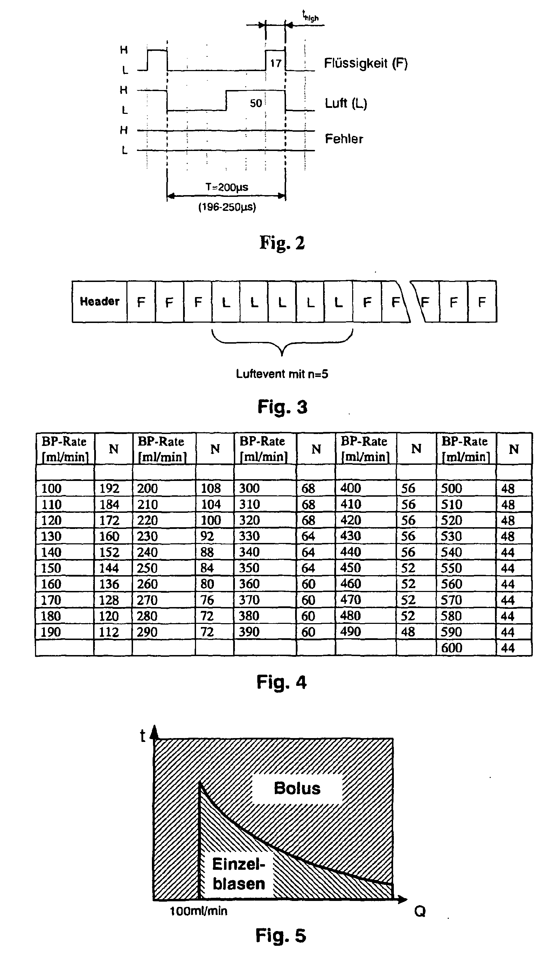

Das Ausgangssignal des Komparators 23 wird der Schaltung 24 zur Pulsweitenmodulation zugeführt. In Abhängigkeit von dem Ausgangssignal des Komparators 23 erzeugt die Schaltung 24 zur Pulsweitenmodulation zwei pulsweitenmodulierte Signale (PWM-Signale). Wenn die maximale Amplitude größer als oder gleich dem Referenzpegel ist, erzeugt die Schaltung 24 ein PWM-Signal mit der Pulsweite T1, während die Schaltung 24 ein PWM-Signal mit der Pulsweite T2 > T1 erzeugt, wenn die Amplitude des Signals kleiner als der vorgegebene Referenzpegel ist. Das PWM-Signal mit der Pulsweite T1 wird nachfolgend mit F (Flüssigkeit) und das PWM-Signal mit der Pulsweite T2 mit L (Luft) bezeichnet (Figur 2).

In dem vorliegenden Ausführungsbeispiel ist nur ein Referenzpegel vorgegeben. Es ist aber auch möglich mehrere Referenzpegel vorzugeben, um zwischen unterschiedlichen Dämpfungen der Signale unterscheiden zu können.

In der Auswerteinheit 21 werden die PWM-Signale blockweise verarbeitet. Da sich der Zyklus der Blutbehandlungsvorrichtung mit T = 50ms deutlich von der zeitlichen Abfolge der Messzyklen (T = 200µs) unterscheidet, werden die PWM-Signale in dem Zwischenspeicher 25 zwischengespeichert und blockweise der Schaltung 26 zur Signalauswertung übergeben, in der die Signalfolge lückenlos erfasst wird.

Die Signalauswertschaltung 26 ist über eine Datenleitung 27 mit der Steuereinheit 15 der Dialysevorrichtung verbunden, die wiederum über die Steuerleitung 16 mit der Blutpumpe 13 verbunden ist. Damit erhält die Auswertschaltung 26 die mit der Blutpumpe 13 vorgegebene Flussrate des durch die Messstrecke 19 fließenden Blutes. Darüber hinaus ist die Signalauswerteschaltung 26 über Signalleitungen 28 und 29 mit einer ersten und zweiten Signaleinrichtung 30 und 31 verbunden, mit denen ein akustischer und/oder optischer Alarm gegeben werden kann. Eine Steuerleitung 32 führt von der Signalauswertschaltung 26 zu der Steuereinheit 15 zur Übertragung eines Steuersignals. Wenn dieses Steuersignal an der Steuereinheit 15 anliegt, unterbricht die Steuereinheit die Blutbehandlung sofort durch Betätigung der venösen Schlauchklemme 33 zum Abklemmen der Blutabführleitung 7 und Anhalten der Blutpumpe 13.

Nachfolgend wird die Funktionsweise der Signalauswertschaltung 26 im einzelnen beschrieben. Die Schaltung 26 weist Mittel 26A auf, um die Anzahl der aufeinanderfolgenden Zeitintervalle zu ermitteln, in denen die maximale Amplitude des Signals kleiner als der vorgegebene Referenzpegel ist, d.h. in dem jeweiligen Zeitintervall das PWM-Signal L vorliegt. Die Signalauswertschaltung ermittelt also die Anzahl der kontinuierlich aufeinanderfolgenden PWM-Signale L. Figur 3 zeigt ein Beispiel für die Signalfolge. In diesem Beispiel folgen 5 PWM-Signale L (n = 5) unmittelbar aufeinander.

Darüber hinaus weist die Signalauswertschaltung 26 Mittel 26B auf, mit denen in Abhängigkeit von der Flussrate des Blutes ein Grenzwert N für die Anzahl n der aufeinanderfolgenden PWM-Signale L festgelegt wird. Die einzelnen Grenzwerte für die einzelnen Flussraten werden empirisch ermittelt.

Figur 4 zeigt eine Tabelle, in der die Grenzwerte N für die Anzahl der aufeinanderfolgenden PWM-Signale L in Abhängigkeit von der Flussrate (BP-Rate) der Blutpumpe 13 abgelegt sind. Die Zuordnung der Werte N zu den einzelnen Flussraten sind in einem Speicher 26C der Signalauswertschaltung 26 gespeichert.

Des weiteren weist die Signalauswertschaltung Mittel 26D zum Vergleichen der ermittelten Anzahl n der aufeinanderfolgenden PWM-Signale L mit dem festgelegten Grenzwert N auf, der dem Speicher 26C entnommen wird, und Mittel 26E zum Erkennen eines ersten Störfalls auf. Der erste Störfall wird dann erkannt, wenn die Anzahl der aufeinanderfolgenden PWM-Signale L größer als der festgelegte Grenzwert N ist. In diesem Fall geben die Mittel zum Erkennen des ersten Störfalls 26E ein Alarmsignal an die erste Alarmeinheit 30 ab, die einen akustischen und/oder optischen Alarm erzeugt. Des weiteren erzeugt die Schaltung 26 ein Steuersignal für die Steuereinheit 15 der Dialysevorrichtung, die sofort die Blutbehandlung unterbricht. Dadurch wird vermieden, dass die von der Auswerteinheit 21 erkannte Luft in den Patienten infundiert wird.

In der Praxis kann es vorkommen, dass aufgrund von Störungen in einer kontinuierlichen Folge von PWM-Signalen L einzelne PWM-Signale F oder auch andere Signale zu finden sind. Diese Störsignale könnten die Signalauswertung stark verfälschen, da beim Auftreten der Störsignale angenommen werden könnte, dass die Signalfolge abgebrochen ist. Daher verfügt die Signalauswertschaltung noch über einen oder mehrere Störfilter, mit denen das Auftreten einzelner PWM-Signale F oder auch anderer Störsignale in einer längeren Folge von PWM-Signalen L erkannt und die Störsignale eliminiert werden. Hierzu werden bekannte Algorithmen zur Fehlererkennung angewandt.

Für den Fall, dass festgestellt wird, dass die Anzahl n der aufeinanderfolgenden PWM-Signale L kleiner oder gleich dem festgelegten Grenzwert N ist, wird darauf geschlossen, dass nicht eine größere Menge an Luft (Bolus) im Blut eingeschlossen ist, sondern nur eine oder mehrere kleine Luftblasen (Einzelblasen) vorhanden sind. Dieses Ereignis wird nicht direkt als Störfall angesehen. Vielmehr wird der Patient erst dann gefährdet, wenn das gesamte Volumen der im Blut eingeschlossenen Luft während der Blutbehandlung einen vorgegebenen Referenzwert überschreitet.

Die Signalauswertschaltung 26 verfügt daher über Mittel 26 F zum Ermitteln der Anzahl der Ereignisse p, bei der die Anzahl n der aufeinanderfolgenden PWM-Signale L kleiner oder gleich dem festgelegten Grenzwert N ist. Des weiteren sind Mittel 26 G zum Vergleichen der Anzahl p dieser Ereignisse mit dem vorgegebenen Referenzwert P und Mittel 26 H zum Erkennen eines zweiten Störfalls vorgesehen. Der zweite Störfall wird dann erkannt, wenn die Anzahl p der Ereignisse größer als der vorgegebene Referenzwert P ist. Als Referenzwert wird in der Praxis ein Wert vorgegeben, der beispielsweise zwischen 8 und 12 liegt. Wenn der zweite Störfall erkannt wird, geben die Mittel zum Erkennen des zweiten Störfalls 26 H ein zweites Alarmsignal an die zweite Alarmeinrichtung 31 ab, die wiederum einen akustischen und/oder optischen Alarm erzeugt, der aber von dem ersten Alarm verschieden ist. Auch in diesem Fall wird ein Steuersignal für die Steuereinheit der Dialysevorrichtung zur Betätigung der venösen Schlauchklemme 33 zum Abklemmen der Blutabführleitung 7 und zum Anhalten der Blutpumpe 13 erzeugt.

Die erfindungsgemäße Überwachungsvorrichtung erlaubt verschiedene Störfälle sicher zu erkennen und zwischen den Störfällen sicher zu unterscheiden, so dass die Sicherheit der Dialysebehandlung weiterhin erhöht wird.

## Patentansprüche

1. Verfahren zum Überwachen einer strömenden Flüssigkeit auf das Vorhandensein von Luft mit folgenden Verfahrensschritten:
Einkoppeln eines Ultraschallsignals mit mindestens einer vorgegebenen Amplitude in die strömende Flüssigkeit,
Empfangen des aus der strömenden Flüssigkeit austretenden Ultraschallsignals,
Vergleichen der Amplitude oder einer mit der Amplitude korrelierenden Größe jedes der in einer kontinuierlichen Folge von Zeitintervallen empfangenen Ultraschallsignale mit einem vorgegebenen Referenzpegel, wobei auf das Vorhandensein eines bestimmten Volumens an Luft in der strömenden Flüssigkeit während des jeweiligen Zeitintervalls geschlossen wird, wenn die Amplitude des Ultraschallsignals kleiner als der vorgegebene Referenzpegel ist, und darauf geschlossen wird, dass das bestimmte Volumen an Luft nicht vorhanden ist, wenn die Amplitude des Ultraschallsignals größer als oder gleich dem Referenzpegel ist,
Ermitteln der Anzahl der aufeinanderfolgenden Zeitintervalle, in denen die Amplitude des empfangenen Ultraschallsignals kleiner als der vorgegebene Referenzpegel ist,
Ermitteln der Flussrate der strömenden Flüssigkeit und Festlegen eines Grenzwertes in Abhängigkeit von der Flussrate, und
Vergleichen der ermittelten Anzahl der aufeinanderfolgenden Zeitintervalle, in denen die Amplitude des empfangenen Ultraschallsignals kleiner als der vorgegebene Referenzpegel ist, mit dem festgelegten Grenzwert, wobei auf einen ersten Störfall geschlossen wird, wenn die Anzahl der aufeinanderfolgenden Zeitintervalle, in denen die Amplitude des empfangenen Ultraschallsignals kleiner als der vorgegebene Referenzpegel ist, größer als der festgelegte Grenzwert ist.

2. Verfahren nach Anspruch 1, **dadurch gekennzeichnet, dass** die Anzahl der Ereignisse ermittelt wird, bei denen die Anzahl der aufeinanderfolgenden Zeitintervalle, in denen die Amplitude des empfangenen Ultraschallsignals kleiner als der vorgegebene Referenzpegel ist, kleiner als oder gleich dem festgelegten Grenzwert ist, wobei die Anzahl der Ereignisse mit einem vorgegebenen Referenzwert verglichen und auf einen zweiten Störfall geschlossen wird, wenn die Anzahl der Ereignisse größer als der vorgegebene Referenzwert ist.

3. Verfahren nach Anspruch 2, **dadurch gekennzeichnet, dass** beim Erkennen des ersten und/oder zweiten Störfalls ein erstes Alarm- und/oder Steuersignal erzeugt wird.

4. Verfahren nach einem der Ansprüche 1 bis 3, **dadurch gekennzeichnet, dass** ein erstes Signal erzeugt wird, wenn die Amplitude des empfangenen Ultraschallsignals kleiner als der vorgegebene Referenzpegel ist und ein zweites Signal erzeugt wird, wenn die Amplitude des empfangenen Ultraschallsignals größer oder gleich dem vorgegebene Referenzpegel ist, wobei die beiden Signale in zeitlicher Abfolge gespeichert werden.

5. Verfahren nach einem der Ansprüche 1 bis 4, **dadurch gekennzeichnet, dass** ein erstes pulsweitenmoduliertes Signal (PWM-Signal) mit einer ersten Pulsweite erzeugt wird, wenn die Amplitude des Ultraschallsignals kleiner als der vorgegebene Referenzpegel ist, und ein zweites pulsweitenmoduliertes Signal (PWM-Signal) erzeugt wird, wenn die Amplitude des Ultraschallsignals größer als oder gleich dem Referenzpegel ist.

6. Verfahren nach Anspruch 4 oder 5, **dadurch gekennzeichnet, dass** die ersten und zweiten Signale zu Signalblöcken zusammengefasst und die Signalblöcke vor der Auswertung zwischengespeichert werden.

7. Verfahren zur extrakorporalen Blutbehandlung mit einem extrakorporalen Blutkreislauf, wobei das Vorhandensein von Luft in dem extrakorporalen Blutkreislauf mit dem Verfahren nach einem der Ansprüche 1 bis 6 überwacht wird.

8. Vorrichtung zum Überwachen einer strömenden Flüssigkeit auf das Vorhandensein von Luft mit:
einem Ultraschall-Sender (17) zum Einkoppeln von einem Ultraschallsignal mit mindestens einer vorgegebenen Amplitude in die strömende Flüssigkeit,
einem Ultraschall-Empfänger (18) zum Empfangen des aus der strömenden Flüssigkeit austretenden Ultraschallsignals, und
einer Auswerteinheit (21), die aufweist:
Mittel (23) zum Vergleichen der Amplitude oder einer mit der Amplitude korrelierenden Größe jedes der in einer kontinuierlichen Folge von Zeitintervallen empfangenen Ultraschallsignale mit einem vorgegebenen Referenzpegel, wobei auf das Vorhandensein eines bestimmten Volumens an Luft in der strömenden Flüssigkeit während des jeweiligen Zeitintervalls geschlossen wird, wenn die Amplitude des Ultraschallsignals kleiner als der vorgegebene Referenzpegel ist, und darauf geschlossen wird, dass das bestimmte Volumen an Luft nicht vorhanden ist, wenn die Amplitude des Ultraschallsignals größer als oder gleich dem Referenzpegel ist,
**gekennzeichnet dadurch, dass** die Auswerteinheit (21) weiterhin aufweist:
Mittel (26A) zum Ermitteln der Anzahl der aufeinanderfolgenden Zeitintervalle, in denen die Amplitude des empfangenen Ultraschallsignals kleiner als der vorgegebene Referenzpegel ist,
Mittel (26B) zum Ermitteln der Flussrate der strömenden Flüssigkeit und Festlegen eines Grenzwertes in Abhängigkeit von der Flussrate,
Mittel (26D) zum Vergleichen der ermittelten Anzahl der aufeinanderfolgenden Zeitintervalle, in denen die Amplitude des empfangenen Ultraschallsignals kleiner als der vorgegebene Referenzpegel ist, mit dem festgelegten Grenzwert, und
Mittel (26E) zum Erkennen eines ersten Störfalls, die derart ausgebildet sind, dass auf den ersten Störfall geschlossen wird, wenn die Anzahl der aufeinanderfolgenden Zeitintervalle, in denen die Amplitude des empfangenen Ultraschallsignals kleiner als der vorgegebene Referenzpegel ist, größer als der festgelegte Grenzwert ist.

9. Vorrichtung nach Anspruch 8, **dadurch gekennzeichnet, dass** die Auswerteinheit (21) ferner aufweist:
Mittel (26F) zum Ermitteln der Anzahl der Ereignisse, bei denen die Anzahl der aufeinanderfolgenden Zeitintervalle, in denen die Amplitude des empfangenen Ultraschallsignals kleiner als der vorgegebene Referenzpegel ist, kleiner als oder gleich dem festgelegten Grenzwert ist,
Mittel (26G) zum Vergleichen der Anzahl der Ereignisse mit einem vorgegebenen Referenzwert, und
Mittel (26H) zum Erkennen eines zweiten Störfalls, die derart ausgebildet sind, dass auf den zweiten Störfall geschlossen wird, wenn die Anzahl der Ereignisse größer als der vorgegebene Referenzwert ist.

10. Vorrichtung nach Anspruch 9, **dadurch gekennzeichnet, dass** die Auswerteinheit (21) ferner aufweist:
Mittel (30, 31) zum Erzeugen eines ersten Alarm- und/oder Steuersignals beim Erkennen des ersten und/oder zweiten Störfalls.

11. Vorrichtung nach einem der Ansprüche 8 bis 10, **dadurch gekennzeichnet, dass** die Auswerteinheit (21) ferner aufweist:
Mittel (24) zum Erzeugen eines ersten Signals, wenn die Amplitude des empfangenen Ultraschallsignals kleiner als der vorgegebene Referenzpegel ist und eines zweiten Signals, wenn die Amplitude des empfangenen Ultraschallsignals größer oder gleich dem vorgegebene Referenzpegel ist, und
Mittel (25) zum Speichern der beiden Signale in zeitlicher Abfolge.

12. Vorrichtung nach einem der Ansprüche 8 bis 11, **dadurch gekennzeichnet, dass** die Auswerteinheit (21) ferner aufweist:
Mittel (24) zum Erzeugen eines ersten PWM-Signals mit einer ersten Pulsweite, wenn die Amplitude des Ultraschallsignals kleiner als der vorgegebene Referenzpegel ist, und Mittel (24) zum Erzeugen eines zweiten PWM-Signals, wenn die Amplitude des Ultraschallsignals größer als oder gleich dem Referenzpegel ist.

13. Vorrichtung nach Anspruch 11 oder 12, **dadurch gekennzeichnet, dass** die Auswerteinheit (21) derart ausgebildet ist, dass die ersten und zweiten Signale zu Signalblöcken zusammengefasst werden und die Auswerteinheit einen Zwischenspeicher (25) aufweist, in dem die Signalblöcke vor der Auswertung zwischengespeichert werden.

14. Vorrichtung zur extrakorporalen Blutbehandlung mit einem extrakorporalen Blutkreislauf und einer Vorrichtung nach einem der Ansprüche 8 bis 13, wobei das Vorhandensein von Luft in dem extrakorporalen Blutkreislauf überwacht wird.

## Claims

1. A method for monitoring a flowing fluid for the presence of air with the following process steps:
coupling of an ultrasound signal with at least one predetermined amplitude into the flowing fluid,
reception of the ultrasound signal emerging from the flowing fluid,
comparison of the amplitude, or a variable correlating with the amplitude, of each of the ultrasound signals received in a continuous sequence of time intervals with a predetermined reference level, it being concluded that there is a presence of a specific volume of air in the flowing fluid during the respective time interval if the amplitude of the ultrasound signal is less than the predetermined reference level, and it being concluded that the specific volume of air is not present if the amplitude of the ultrasound signal is greater than or equal to the reference level,
determination of the number of successive time intervals in which the amplitude of the received ultrasound signal is less than the predetermined reference level,
determination of the flow rate of the flowing fluid and fixing of a threshold value as a function of the flow rate, and
comparison of the ascertained number of successive time intervals, in which the amplitude of the received ultrasound signal is less than the predetermined reference level, with the fixed threshold value, it being concluded that there is a first malfunction if the number of successive time intervals, in which the amplitude of the received ultrasound signal is less than the predetermined threshold level, is greater than the fixed threshold value.

2. The method according to claim 1, **characterised in that** the number of events is ascertained in which the number of successive time intervals, in which the amplitude of the received ultrasound signal is less than the predetermined reference level, is less than or equal to the fixed threshold value, the number of events being compared with a predetermined reference value and it being concluded that there is a second malfunction if the number of events is greater than the predetermined reference value.

3. The method according to claim 2, **characterised in that** a first alarm signal and/or control signal is generated when the first and/or second malfunction is detected.

4. The method according to any one of claims 1 to 3, **characterised in that** a first signal is generated if the amplitude of the received ultrasound signal is less than the predetermined reference level and a second signal is generated if the amplitude of the received ultrasound signal is greater than or equal to the predetermined reference level, the two signals being stored in a time sequence.

5. The method according to any one of claims 1 to 4, **characterised in that** a first pulse-width modulated signal (PWM signal) with a first pulse width is generated if the amplitude of the ultrasound signal is less than the predetermined reference level, and a second pulse-width modulated signal (PWM signal) is generated if the amplitude of the ultrasound signal is greater than or equal to the reference level.

6. The method according to claim 4 or 5, **characterised in that** the first and second signals are combined into signal blocks and the signal blocks are temporarily stored before evaluation.

7. A method for extracorporeal blood treatment with an extracorporeal blood circuit, wherein the presence of air in the extracorporeal blood circuit is monitored by the method according to any one of claims 1 to 6.

8. A device for monitoring a flowing fluid for the presence of air with:
an ultrasound transmitter (17) for coupling an ultrasound signal with at least one predetermined amplitude into the flowing fluid,
an ultrasound receiver (18) for receiving the ultrasound signal emerging from the flowing fluid, and
an evaluation unit (21), which comprises:
means (33) for comparing the amplitude, or a variable correlating with the amplitude, of each of the ultrasound signals received in a continuous sequence of time intervals with a predetermined reference level, it being concluded that there is the presence of a specific volume of air in the flowing fluid during the respective time interval if the amplitude of the ultrasound signal is smaller than the predetermined reference level, and it being concluded that the specific volume of air is not present if the amplitude of the ultrasound signal is greater than or equal to the reference level,
**characterised in that** the evaluation unit (21) further comprises:
means (26A) for determining the number of successive time intervals in which the amplitude of the received ultrasound signal is less than the predetermined reference level,
means (26B) for determining the flow rate of the flowing fluid and fixing a threshold value as a function of the flow rate,
means (26D) for comparing the determined number of successive time intervals, in which the amplitude of the received ultrasound signal is less than the predetermined reference level, with the fixed threshold value, and
means (26E) for detecting a first malfunction, which are constituted such that it is concluded that there is a first malfunction if the number of successive time intervals, in which the amplitude of the received ultrasound signal is less than the predetermined reference level, is greater than the fixed threshold value.

9. The device according to claim 8, **characterised in that** the evaluation unit (21) further comprises:
means (26F) for determining the number of events in which the number of successive time intervals, in which the amplitude of the received ultrasound signal is less than the predetermined reference level, is less than or equal to the fixed threshold value,
means (26G) for comparing the number of events with a predetermined reference value, and
means (26H) for detecting a second threshold value, which are constituted such that it is concluded that there is a second malfunction if the number of events is greater than the predetermined reference value.

10. The device according to claim 9, **characterised in that** the evaluation unit (21) further comprises:
means (30, 31) for generating a first alarm signal and/or control signal when the first and/or second malfunction is detected.

11. The device according to any one of claims 8 to 10, **characterised in that** the evaluation unit (21) further comprises:
means (24) for generating a first signal if the amplitude of the received ultrasound signal is less than the predetermined reference level and a second signal if the amplitude of the received ultrasound signal is greater than or equal to the predetermined reference level, and means (25) for storing the two signals in a time sequence.

12. The device according to any one of claims 8 to 11, **characterised in that** the evaluation unit (21) further comprises:
means (24) for generating a first PWM signal with a first pulse width if the amplitude of the ultrasound signal is less than the predetermined reference level,
and means (24) for generating a second PWM signal if the attitude of the ultrasound signal is greater than or equal to the reference level.

13. The device according to claim 11 or 12, **characterised in that** the evaluation unit (21) is constituted such that the first and second signals are combined into signal blocks and the evaluation unit comprises a temporary memory (25), in which the signal blocks are temporarily stored before evaluation.

14. An apparatus for extracorporeal blood treatment with an extracorporeal blood circuit and the device according to any one of claims 8 to 13, the presence of air in the extracorporeal blood circuit being monitored.

## Revendications

1. Procédé pour surveiller la présence d'air dans un écoulement liquide, comportant les étapes suivantes :
- couplage d'un signal ultrasonore ayant au moins une amplitude prescrite à l'écoulement liquide,
- réception du signal ultrasonore sortant de l'écoulement liquide,
- comparaison de l'amplitude ou d'une grandeur corrélée à l'amplitude de chacun des signaux ultrasonores reçus pendant une séquence continue d'intervalles temporels, avec un niveau de référence prescrit, permettant de conclure à la présence d'un volume d'air défini dans l'écoulement liquide pendant l'intervalle temporel correspondant, lorsque l'amplitude du signal ultrasonore est inférieure au niveau de référence prescrit, et de conclure en conséquence à l'absence du volume d'air défini, lorsque l'amplitude du signal ultrasonore est supérieure ou égale au niveau de référence,
- détermination du nombre d'intervalles tempo-rels successifs pendant lesquels l'amplitude du signal ultrasonore reçu est inférieure au niveau de référence prescrit,
- détermination du débit de l'écoulement liquide et définition d'une valeur limite fonction du débit d'écoulement, et
- comparaison du nombre d'intervalles temporels successifs déterminé pendant lesquels l'ampli-tude du signal ultrasonore reçu est inférieure au niveau de référence prescrit, avec la valeur limite définie, permettant de conclure à un premier incident lorsque le nombre des intervalles temporels successifs pendant lesquels l'amplitude du signal ultrasonore reçu est inférieure au niveau de référence prescrit, est supérieur à la valeur limite définie.

2. Procédé selon la revendication 1, **caractérisé en ce qu'**il est procédé à une détermination du nombre d'événements pour lesquels le nombre des intervalles temporels successifs pendant lesquels l'amplitude du signal ultrasonore reçu est inférieure au niveau de référence prescrit, est inférieur ou égal à la valeur limite définie, le nombre d'événements étant comparé à une valeur de référence prescrite, permettant de conclure à un deuxième incident lorsque le nombre des incidents est supérieur à la valeur de référence prescrite.

3. Procédé selon la revendication 2, **caractérisé en ce qu'**un premier signal d'alarme et/ou de commande est généré à la détection du premier et/ou du deuxième incident.

4. Procédé selon l'une des revendications 1 à 3, **caractérisé en ce qu'**un premier signal est généré lorsque l'amplitude du signal ultrasonore reçu est inférieure au niveau de référence prescrit, et un deuxième signal généré lorsque l'amplitude du signal ultrasonore reçu est supérieure ou égale au niveau de référence prescrit, les deux signaux étant mémorisés en une séquence temporelle.

5. Procédé selon l'une des revendications 1 à 4, **caractérisé en ce qu'**un premier signal modulé en largeur d'impulsion (signal PWM) est généré avec une première largeur d'impulsion lorsque l'ampli-tude du signal ultrasonore est inférieure au niveau de référence prescrit, et un deuxième signal modulé en largeur d'impulsion (signal PWM) généré lorsque l'amplitude du signal ultrasonore est supérieure ou égale au niveau de référence.

6. Procédé selon la revendication 4 ou la revendication 5, **caractérisé en ce que** les premiers et deuxièmes signaux sont regroupés en blocs de signaux, et **en ce que** les blocs de signaux sont temporairement mémorisés avant évaluation.

7. Procédé de traitement sanguin extracorporel avec un circuit sanguin extracorporel, la pré-sence d'air dans le circuit sanguin extracorporel étant surveillée au moyen du procédé selon l'une des revendications 1 à 6.

8. Dispositif pour surveiller la présence d'air dans un écoulement liquide, comportant:
- un émetteur ultrasonore (17) pour le couplage d'un signal ultrasonore ayant au moins une amplitude prescrite à l'écoulement liquide,
- un récepteur ultrasonore (18) pour la récep-tion du signal ultrasonore sortant de l'écoulement liquide, et
- une unité d'évaluation (21) comprenant:
- des moyens (23) pour comparer l'amplitude ou une grandeur corrélée à l'amplitude de chacun des signaux ultrasonores reçus pendant une séquence continue d'inter-valles temporels, avec un niveau de réfé-rence prescrit, permettant de conclure à la présence d'un volume d'air défini dans l'écoulement liquide pendant l'intervalle temporel correspondant, lorsque l'ampli-tude du signal ultrasonore est inférieure au niveau de référence prescrit, et de conclure en conséquence à l'absence du volume d'air défini, lorsque l'amplitude du signal ultrasonore est supérieure ou égale au niveau de référence,
- des moyens (26A) pour déterminer le nombre d'intervalles temporels successifs pendant lesquels l'amplitude du signal ultrasonore reçu est inférieure au niveau de référence prescrit,
- des moyens (26B) pour déterminer le débit de l'écoulement liquide et définir une valeur limite fonction du débit d'écoule-ment, et
- des moyens (26D) pour comparer le nombre d'intervalles temporels successifs déter-miné pendant lesquels l'amplitude du signal ultrasonore reçu est inférieure au niveau de référence prescrit, avec la valeur limite définie, et
- des moyens (26E) pour détecter un premier incident, prévus pour permettre de conclure au premier incident lorsque le nombre des intervalles temporels successifs pendant lesquels l'ampli-tude du signal ultrasonore reçu est inférieure au niveau de référence prescrit, est supérieur à la valeur limite définie.

9. Dispositif selon la revendication 8, caracté-risé en ce que l'unité d'évaluation (21) comprend en outre:
- des moyens (26F) pour déterminer le nombre des événements pour lesquels le nombre des intervalles temporels successifs pendant lesquels l'amplitude du signal ultrasonore reçu est inférieure au niveau de référence prescrit, est inférieur ou égal à la valeur limite définie,
- des moyens (26G) pour comparer le nombre d'événements à une valeur de référence prescrite, et
- des moyens (26H) pour détecter un deuxième incident, prévus pour permettre de conclure à un deuxième incident lorsque le nombre des incidents est supérieur à la valeur de référence prescrite.

10. Dispositif selon la revendication 9, carac-térisé en ce que l'unité d'évaluation (21) comprend en outre:
- des moyens (30, 31) pour générer un premier signal d'alarme et/ou de commande à la détec-tion du premier et/ou du deuxième incident.

11. Dispositif selon l'une des revendications 8 à 10, **caractérisé en ce que** l'unité d'évaluation (21) comprend en outre:
- des moyens (24) pour générer un premier signal lorsque l'amplitude du signal ultrasonore reçu est inférieure au niveau de référence pres-crit, et un deuxième signal lorsque l'ampli-tude du signal ultrasonore reçu est supérieure ou égale au niveau de référence prescrit, et des moyens (25) pour mémoriser les deux signaux en une séquence temporelle.

12. Dispositif selon l'une des revendications 8 à 11, **caractérisé en ce que** l'unité d'évaluation (21) comprend en outre:
- des moyens (24) pour générer un premier signal PWM avec une première largeur d'impulsion lorsque l'amplitude du signal ultrasonore est inférieure au niveau de référence prescrit, et des moyens (24) pour générer un deuxième signal PWM lorsque l'amplitude du signal ultrasonore est supérieure ou égale au niveau de référence.

13. Dispositif selon la revendication 11 ou la revendication 12, **caractérisé en ce que** l'unité d'évaluation (21) est réalisée de telle manière que les premiers et deuxièmes signaux sont regroupés en blocs de signaux, et **en ce que** l'unité d'évaluation comporte une mémoire tampon (25), où les blocs de signaux sont temporairement mémorisés avant évaluation.

14. Dispositif de traitement sanguin extracorporel avec un circuit sanguin extracorporel et un dispositif selon l'une des revendications 8 à 13, où la présence d'air est surveillée dans le circuit sanguin extracorporel.
